# EUROPEAN PATENT SPECIFICATION

(11) **EP 4 109 458 B1**
(45) Date of publication and mention of the grant of the patent: **25.11.2026**
(21) Application number: 21756980.5
(22) Date of filing: 29.01.2021
(51) Int. Cl.: G16H 20/30, A61B 5/11, A61B 5/00, G16Y 10/60, A61B 5/16, G06N 3/08, G06N 3/09, G16H 50/70, G16H 50/30, G16H 50/20, G06N 3/0499, G16H 20/70

(54) **OCCUPATIONAL THERAPY SUPPORTING DEVICE**
ERGOTHERAPIEUNTERSTÜTZUNGSVORRICHTUNG
DISPOSITIF DE PRISE EN CHARGE D'ERGOTHÉRAPIE

(30) Priority: 18.02.2020 JP 2020024812
(43) Date of publication of application: 28.12.2022
(73) Proprietor: Rehabilitation3.0 Co., Ltd., Osaka 590-0952 (JP)
(72) Inventor: MASUDA Hirokazu, Sakai-shi, Osaka 590-0952 (JP)
(74) Representative: Rebbereh, Cornelia
(86) International application number: PCT/JP2021/003400
(87) International publication number: WO 2021/166605

(56) References cited:
- WO-A1-2016/170005
- WO-A1-2019/005039
- WO-A2-2018/151173
- JP-A- 2009 288 933
- JP-A- 2010 020 446
- JP-A- 2019 057 282
- JP-A- 2020 006 836
- US-A1- 2017 164 893
- US-A1- 2019 065 970
- US-A1- 2021 321 883
- CONCI JASON ET AL: "Utilizing Consumer-grade Wearable Sensors for Unobtrusive Rehabilitation Outcome Prediction", 2019 IEEE EMBS INTERNATIONAL CONFERENCE ON BIOMEDICAL & HEALTH INFORMATICS (BHI), IEEE, 19 May 2019 (2019-05-19), pages 1 - 4, XP033614272, [retrieved on 20190911], DOI: 10.1109/BHI.2019.8834512
- ANONYMOUS: "Developed a rehabilitation support cloud system for day service providers", PANASONIC PRESS RELEASE, 25 September 2019 (2019-09-25), XP055849743, Retrieved from the Internet <URL:https://news.panasonic.com/jp/press/data/2019/09/jn190925-1/jn190925-1.pdf>

## Description

### TECHNICAL FIELD

The present invention relates to an occupational therapy support device that supports occupational therapy.

### BACKGROUND ART

About 55 years have passed since occupational therapy was legalized in 1965 (Showa 40). During this period, it is said that occupational therapy and occupational therapists have developed and grown as professionals who can contribute to the health condition of the people in various fields such as health, medical, and welfare while responding to changes in social structures and changes in people's awareness of health and disability.

"Occupational therapy" means providing treatment, guidance, and assistance to a person with a physical or mental disorder or a person who is predicted to have a physical or mental disorder, by using work activities that encourage recovery, maintenance, and development of various functions, in order to allow the person to acquire an independent life (definition based on the practice of "occupational therapy" by the Japan Association of Occupational Therapists). The term "occupational" refers to all activities about humans, such as various actions in daily life, work, and play, and means treatment, assistance, or guidance (definition of "occupational" by the Japan Occupational Therapists Association). When performing occupational therapy, a qualified occupational therapist explains first occupational therapy evaluation (assessment) in an interview with the person subjected to prescription and his/her family and performs the occupational therapy evaluation after obtaining consent. In order to evaluate the occupational therapy, the therapist performs information collection from a medical chart and the like, an interview, behavior observation, examination, measurement, and the like. The therapist then prepares an occupational therapy plan and executes the occupational therapy based on the plan. In addition, during therapy, the occupational therapy evaluation is periodically performed, and the effect of the occupational therapy is measured. (For information refer to Non-Patent Literature 1.)

As an effective means for evaluating occupational therapy, functional independence measure (FIM) is known. FIM has been developed in the United States as a method of evaluating how much a person can perform activity of daily living (ADL) by himself/herself. Since the degree of care burden can be particularly evaluated by evaluating activities of daily living by FIM, FIM is a well-known method widely used in the field of rehabilitation and the like also in our country. FIM includes a total of 18 items of movement items and cognitive items, and each item is evaluated in seven levels of 1 point to 7 points. The cognitive items include five items, namely, comprehension, expression, social interaction, problem solving, and memory. The exercise items include 13 items in total, such as eating, grooming, wiping, dressing, going to the bathroom, urination management, defecation management, transfer (sitting-up), and locomotion.

Although FIM is said to be an evaluation method that allows anyone to perform measurement, there is a problem that evaluation results are sometimes inconsistent because of personal judgments by evaluators. When qualified occupational therapists perform evaluation, the variation in evaluation is small. However, in particular, when persons who are not experts in movement perform evaluation, a problem arises. Note that Patent Literature 1 discloses a sleep state determination device that determines a sleep state on the basis of vital data of a user during sleep and subjective data of the user with respect to sleep.

### CITATIONS LIST

### PATENT LITERATURE

Patent Literature 1: JP 2019-068907 A

### NON PATENT LITERATURE

Non Patent Literature 1: "Occupational Therapy Guidelines (2012 version)", Japan Association of Occupational Therapists (http://www.jaot.or.jp/wp-content/uploads/2013/08/OTguideline-2012.pdf)
WO 2016/170005 A1 discloses a system to identify a person for monitoring physiological parameters of one or more persons, the system including: one or more sensors for monitoring the one or more persons' physiological parameters, and one or more processors, the one or more processors configured to process signals from the one or more sensors to identify a person, the processing further comprising an evaluation of features comprising one or more respiratory features, cardiac features, or movement features.
US 2019/0065970 A1 discloses a monitoring system, comprising at least one monitor device configured to monitor one or more physical properties of a user; an artificial intelligence (AI) system configured to receive and analyze the monitored physical properties to generate one or more activity parameters optimized or personalized to the user.
Document CONCI JASON ET AL: "Utilizing Consumer-grade Wearable Sensors for Unobtrusive Rehabilitation Outcome Prediction",2019 IEEE EMBS INTERNATIONAL CONFERENCE ON BIOMEDICAL & HEALTH INFORMATICS (BHI), IEEE, 19 May 2019 (2019-05-19), pages 1-4, discloses that an improved prediction accuracy can be provided by use of consumer-grade fitness devices to obtain highly accurate FIM. Physical activity, heart rate, and sleep features are extracted to use as input to machine learning models which are trained to predict FIM.

### SUMMARY OF INVENTION

### TECHNICAL PROBLEMS

The present invention has been made in view of the above problems and has its object to provide an occupational therapy support device that enables a user to obtain data useful for the implementation of occupational therapy and independent of an evaluator. Another object of the present invention is to provide a training device that trains an artificial intelligence for the occupational therapy support device. A still another object of the present invention is to provide a method of using the occupational therapy support device.

### SOLUTIONS TO PROBLEMS

The problems are solved by an occupational therapy support device according to claim 1. In order to achieve the above object, a device according to the first aspect of the present invention is an occupational therapy support device including an input data reception unit, an estimation unit, and an estimated data output unit. The input data reception unit receives the input of input data including sleep data that is data about the sleep of a subject of occupational therapy and basic data that is data about the body of the subject. The estimation unit inputs the input data whose input has been received by the input data reception unit to a trained artificial intelligence to cause the artificial intelligence to compute the estimated data of data including activity of daily living data that is data about the activity of daily living of the subject. The estimated data output unit outputs the estimated data computed by the artificial intelligence.

According to this configuration, on the basis of sleep data such as the number of turns of the subject of occupational therapy, for example, and basic data such as the age and height of the subject, for example, the estimated data of data regarding activities of daily living such as eating and going to the bathroom, which is useful for occupational therapy evaluation, is obtained. The sleep data and the basic data, which are input data, are objective data that do not depend on the evaluator. Accordingly, data useful for occupational therapy evaluation that is independent of the evaluator is obtained. Note that the trained artificial intelligence may be part of the occupational therapy support device having this configuration or may be an external device such as one placed on an external cloud server.

A device according to the second aspect of the present invention is an occupational therapy support device including an input data reception unit, a first estimation unit, a second estimation unit, and an estimated data output unit. The input data reception unit receives the input of input data including sleep data that is data about the sleep of a subject of occupational therapy evaluation and basic data that is data about the body of the subject. The first estimation unit inputs the input data whose input has been received by the input data reception unit to a trained first artificial intelligence to cause the first artificial intelligence to compute the estimated data of data including activity of daily living data that is data about the activity of daily living of the subject. The second estimation unit inputs the estimated data computed by the first artificial intelligence to a trained second artificial intelligence to cause the second artificial intelligence to compute the estimated data of the prescription data of the occupational therapy for the subject. The estimated data output unit outputs the estimated data of the prescription data computed by the second artificial intelligence. The prescription data includes data expressing the contents to be prescribed for at least one item of movement, massage, stretching, and a bedding condition.

According to this configuration, on the basis of sleep data such as the number of turns of the subject of occupational therapy, for example, and basic data such as the age and height of the subject, for example, the estimated data of data regarding activities of daily living such as eating and going to the bathroom, which is useful for occupational therapy evaluation, is computed. The sleep data and the basic data, which are input data, are objective data that do not depend on the evaluator. Therefore, the computed estimated data is objective data that does not depend on the evaluator. Furthermore, since the estimated data of the prescription data of occupational therapy is obtained based on the computed estimated data, the data can be useful for planning an occupational therapy plan. Since basic data is objective data that does not depend on the evaluator, the obtained prescription data is also objective data that does not depend on the evaluator. That is, this configuration provides objective data that does not depend on the evaluator and is useful for making an occupational therapy plan. Note that the trained artificial intelligence may be part of the occupational therapy support device having this configuration or may be an external device such as one placed on an external cloud server.

The device according to the third aspect of the present invention is the occupational therapy support device according to the second aspect, in which the prescription data further includes data expressing a movement instruction for at least one item of activity of daily living included in the activity of daily living data.

According to this configuration, it is possible to additionally use a movement instruction regarding at least one item among items regarding the activity of daily living, such as "suppress the water intake" regarding eating, to make an occupational therapy plan. Since basic data is objective data that does not depend on the evaluator, the obtained data expressing a movement instruction is also objective data that does not depend on the evaluator.

The device according to the fourth aspect of the present invention is the occupational therapy support device according to any one of the first to third aspects, in which the sleep data is the data of items regarding sleep and including the number of turns, the number of body movements, the number of times of going to the bathroom, and the time in the bathroom. The basic data is data of basic items including age, height, weight, and medical history. The activity of daily living data is data of items related to daily activities including eating, going to the bathroom, defecation, transfer to the bathroom, locomotion/walk, comprehension, problem solving, and memory.

According to this configuration, the estimated data of the activity of daily living data including items sufficient for performing occupational therapy evaluation is computed based on the sleep data and the basic data of the subject of the occupational therapy evaluation including the minimum items.

The device according to the fifth aspect of the present invention is the occupational therapy support device according to any one of the first to fourth aspects, in which the data including the activity of daily living data further includes physical function data that is data regarding a physical function of the subject.

According to this configuration, not only the estimated data of the activity of daily living data but also the estimated data of the data regarding physical functions such as grip strength are calculated, and hence it is possible to obtain data useful for occupational therapy evaluation or occupational therapy planning with higher accuracy. Since the sleep data and the basic data, which are input data, are objective data that do not depend on the evaluator, the estimated data of the physical function data is also objective data that does not depend on the evaluator.

The device according to the sixth aspect of the invention is the occupational therapy support device according to any one of the first to fifth aspects, in which the activity of daily living data includes a fall risk which is a possibility of falling.

According to this configuration, the estimated data of the activity of daily living data including a fall risk is computed, and hence it is possible to obtain data useful for occupational therapy evaluation or occupational therapy planning with higher accuracy.

The device according to the seventh aspect of the present invention is the occupational therapy support device according to any one of the first to sixth aspects, in which the sleep data further includes an answer to a question about sleep from the subject.

According to this configuration, the sleep data includes subjective evaluation data of the subject such as "good sleep", and hence it is possible to obtain data useful for occupational therapy evaluation or occupational therapy planning with higher accuracy. In addition, since the subjective evaluation data of the subject does not depend on the evaluator, this configuration does not hinder the acquisition of objective data which does not depend on the evaluator and is useful for occupational therapy evaluation or occupational therapy planning.

The device according to the eighth aspect of the present invention is the occupational therapy support device according to any one of the first to seventh aspects, in which the input data further includes environment data that is data regarding an environment of the subject during sleep.

According to this configuration, the input data includes environment data of the subject such as illuminance during sleep, and hence it is possible to obtain data useful for occupational therapy evaluation or occupational therapy planning with higher accuracy. In addition, since the environment data of the subject does not depend on the evaluator, this configuration does not hinder the acquisition of objective data which does not depend on the evaluator and is useful for occupational therapy evaluation or occupational therapy planning.

The device according to the ninth aspect of the present invention is the occupational therapy support device according to any one of the first to eighth aspects, in which the input data includes data including the sleep data of the subject and the basic data of the subject at a plurality of time points from a present to a past in association with time data of the plurality of corresponding time points.

According to this configuration, it is possible to obtain data useful for the occupational therapy evaluation or occupational therapy planning with higher accuracy in consideration of the history of the sleep data, basic data, and the like of the subject of the occupational therapy evaluation.

A device according to the 10th aspect of the present invention is an artificial intelligence training device for an occupational therapy support device and includes an input data reception unit, a training data reception unit, and a training unit. The input data reception unit receives the input of input data including sleep data that is data about the sleep of a subject of occupational therapy evaluation and basic data that is data about the body of the subject. The training data reception unit receives the input of training data that is data corresponding to the input data and including activity of daily living data that is data regarding activity of daily living of the subject. The training unit inputs the input data whose input has been received by the input data reception unit and the training data whose input has been received by the training data reception unit to an artificial intelligence to train the artificial intelligence so as to estimate the training data from the input data.

According to this configuration, the artificial intelligence that can be used for the occupational therapy support device according to the first aspect is constructed by training. Note that the artificial intelligence may be part of the artificial intelligence training device having this configuration or may be an external device such as one placed on an external cloud server.

The device according to the 11th aspect of the present invention is the artificial intelligence training device for the occupational therapy support device according to the 10th aspects, in which the sleep data is the data of items regarding sleep and including the number of turns, the number of body movements, the number of times of going to the bathroom, and the time in the bathroom. The basic data is data of basic items including age, height, weight, and medical history. The activity of daily living data is data of items related to daily activities including eating, going to the bathroom, defecation, transfer to the bathroom, locomotion/walk, comprehension, problem solving, and memory.

According to this configuration, an artificial intelligence which can be used to compute the estimated data of data including the activity of daily living data of the subject in the occupational therapy support device according to the fourth aspect is constructed by training.

The device according to the 12th aspect of the present invention is the artificial intelligence training device for an occupational therapy support device according to the 10th or 11th aspect, in which the data including the activity of daily living data further includes physical function data that is data regarding a physical function of the subject.

According to this configuration, an artificial intelligence which can be used to compute the estimated data of data including the activity of daily living data of the subject in the occupational therapy support device according to the fifth aspect is constructed by training.

The device according to the 13th aspect of the invention is the artificial intelligence training device for an occupational therapy support device according to any one of the 10th to 12th aspects, in which the activity of daily living data includes a fall risk which is a possibility of falling.

According to this configuration, an artificial intelligence which can be used to compute the estimated data of data including the activity of daily living data of the subject in the occupational therapy support device according to the sixth aspect is constructed by training.

The device according to 14th aspect of the present invention is the artificial intelligence training device for an occupational therapy support device according to any one of the 10th to 13th aspects, in which the sleep data further includes an answer to a question about sleep from the subject.

According to this configuration, an artificial intelligence which can be used to compute the estimated data of data including the activity of daily living data of the subject in the occupational therapy support device according to the seventh aspect is constructed by training.

The device according to the 15th aspect of the present invention is the artificial intelligence training device for an occupational therapy support device according to any one of the 10th to 14th aspects, in which the input data further includes environment data that is data regarding an environment of the subject during sleep.

According to this configuration, an artificial intelligence which can be used to compute the estimated data of data including the activity of daily living data of the subject in the occupational therapy support device according to the eighth aspect is constructed by training.

The device according to the 16th aspect of the present invention is the artificial intelligence training device for an occupational therapy support device according to any one of the 10th to 15th aspects, in which the input data includes data including the sleep data of the subject and the basic data of the subject at a plurality of time points from a present to a past in association with time data of the plurality of corresponding time points.

According to this configuration, an artificial intelligence which can be used to compute the estimated data of data including the activity of daily living data of the subject in the occupational therapy support device according to the ninth aspect is constructed by training.

A device according to the 17th aspect of the present invention is an artificial intelligence training device for an occupational therapy support device and includes an input data reception unit, a training data reception unit, and a training unit. The input data reception unit receives the input of input data including activity of daily living data that is data regarding activity of daily living of the subject of occupational therapy evaluation. The training data reception unit receives the input of training data which corresponds to the input data and is prescription data of occupational therapy for the subject. The training unit inputs the input data whose input has been received by the input data reception unit and the training data whose input has been received by the training data reception unit to an artificial intelligence to train the artificial intelligence so as to estimate the training data from the input data. The prescription data includes data expressing the contents to be prescribed for at least one item of movement, massage, stretching, and a bedding condition.

According to this configuration, artificial intelligence that can be used as the second artificial intelligence in the occupational therapy support device according to the second aspect is constructed by training. Note that the artificial intelligence may be part of the artificial intelligence training device having this configuration or may be an external device such as one placed on an external cloud server.

The device according to the 18 the aspect of the present invention is the artificial intelligence training device for an occupational therapy support device according to the 17th aspect, in which the activity of daily living data is data of items regarding daily activities, including eating, going to the bathroom, defecation, transfer to the bathroom, locomotion/walk, comprehension, problem solving, and memory.

According to this configuration, an artificial intelligence that can be used as the second artificial intelligence in the occupational therapy support device according to the fourth aspect is constructed by training.

The device according to the 19th aspect of the present invention is the artificial intelligence training device for the occupational therapy support device according to the 17th or 18th aspect, in which the prescription data further includes data expressing a movement instruction for at least one item of activity of daily living included in the activity of daily living data.

According to this configuration, an artificial intelligence that can be used as the second artificial intelligence in the occupational therapy support device according to the third aspect is constructed by training.

The device according to the 20th aspect of the present invention is the artificial intelligence training device for an occupational therapy support device according to any one of the 17th to 19th aspects, in which the input data further includes physical function data that is data regarding a physical function of the subject.

According to this configuration, an artificial intelligence that can be used as the second artificial intelligence in the occupational therapy support device according to the fifth aspect is constructed by training.

The device according to the 21st aspect of the invention is the artificial intelligence training device for an occupational therapy support device according to any one of the 17th to 20th aspects, wherein the activity of daily living data includes a fall risk which is a possibility of falling.

According to this configuration, an artificial intelligence that can be used as the second artificial intelligence in the occupational therapy support device according to the sixth aspect is constructed by training.

A method according to the 22nd aspect of the present invention is a method of using the occupational therapy support device according to any one of the first to ninth aspects, the method including (a) inputting the input data to the occupational therapy support device, (b) making the estimated data output unit of the occupational therapy support device output the estimated data, and (c) performing occupational therapy based on the output estimated data.

According to this configuration, a user can perform occupational therapy on the basis of objective estimated data independent of the evaluator.

### ADVANTAGEOUS EFFECTS OF INVENTION

As described above, the present invention implements an occupational therapy support device that enables a user to obtain data useful for the implementation of occupational therapy and independent of an evaluator. In addition, the present invention implements a training device that trains an artificial intelligence for the occupational therapy support device. Furthermore, the present invention implements a method of using the occupational therapy support device.

Objects, features, aspects, and advantages of the present invention will become more apparent from the following detailed description and the accompanying drawings.

### BRIEF DESCRIPTION OF DRAWINGS

FIG. 1 is a diagram illustrating the configuration of an occupational therapy support system including an occupational therapy support device according to an embodiment of the present invention.
FIG. 2 is a block diagram illustrating the configuration of the occupational therapy support device in FIG. 1.
FIGS. 3A to 3D are tabular diagrams illustrating input data and output data of the occupational therapy support device illustrated in FIG. 2.
FIG. 4 is a schematic diagram illustrating the conceptual configuration of an artificial intelligence of the occupational therapy support device illustrated in FIG. 2.
FIG. 5 is a block diagram illustrating the configuration of an occupational therapy support device according to another embodiment of the invention.
FIG. 6 is a tabular diagram illustrating output data of the occupational therapy support device illustrated in FIG. 5.

### DESCRIPTION OF EMBODIMENTS

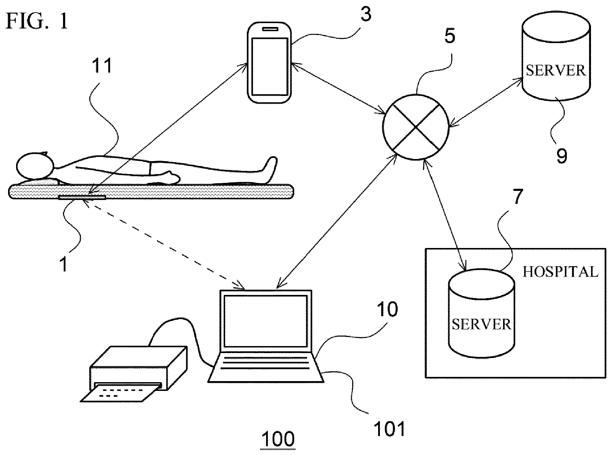

FIG. 1 is a diagram illustrating the configuration of an occupational therapy support system including an occupational therapy support device according to an embodiment of the present invention. An occupational therapy support system 100 includes a sleep sensor 1, a user communication terminal 3, a network 5, and servers 7 and 9 in addition to an occupational therapy support device 101. The sleep sensor 1, the user communication terminal 3, the network 5, and the servers 7 and 9 are devices that are directly or indirectly connected to the occupational therapy support device 101 and cooperate with the occupational therapy support device 101.

The occupational therapy support device 101 is a device that supports occupational therapy by outputting activity of daily living data and the estimated data of physical function data for performing occupational therapy evaluation based on the sleep data and the basic data of a subject 11 of the occupational therapy evaluation. The occupational therapy support device 101 is incorporated in the computer 10 in the illustrated example. That is, installing and activating a specific application in the computer 10 causes a processing device (processor) such as a central processing unit (CPU) of the computer 10 to function as the occupational therapy support device 101.

The sleep sensor 1 is a sensor that automatically acquires the sleep data of the subject 11 of occupational therapy evaluation (hereinafter abbreviated as "subject") and has a communication function of transmitting the acquired data to the user communication terminal 3 or the occupational therapy support device 101 by radio or the like. The sleep data is data about the sleep of the subject 11, such as the sleeping time, the number of turns, respiration during sleep, and pulse. In the illustrated example, the sleep sensor 1 is a mat-shaped sensor that is used while being placed under the bedding on which the subject 11 lies. The sleep sensor 1 in this form is also already commercially available and well known. As the sleep sensor 1, a sleep sensor that can simultaneously collect environment data during sleep, such as a room temperature, humidity, and illuminance is also known. Each item of sleep data and environment data will be described later.

In the illustrated example, the user communication terminal 3 is a smartphone owned by the user. The user is, for example, the subject 11 himself/herself or a relative who takes care of the subject 11. Installing a specific application in advance in the user communication terminal 3 allows the user communication terminal 3 to communicate with the sleep sensor 1. In communication, it is possible to prevent information leakage by, for example, requiring the input of an identification code (ID) and a password. The measurement data received by the user communication terminal 3 from the sleep sensor 1 is transmitted to the occupational therapy support device 101 via the network 5. Even in communication between the user communication terminal 3 and the occupational therapy support device 101, it is possible to prevent information leakage by, for example, requiring the input of an identification code (ID) and a password. The user can also input, to the user communication terminal 3, subjective evaluation data regarding the sleep of the subject 11, for example, a selection result regarding sleepiness namely "had a good sleep", "could not say either", or "did not sleep". The user can also photograph the bedding state of the subject 11 and transmit the bedding state as one of environment data to the occupational therapy support device 101 using the user communication terminal 3. For example, when an application installed in the user communication terminal 3 is activated, a question or an instruction is displayed, and the subjective evaluation data and the environment data can be input or photographed in a format responding to the question or instruction.

The network 5 is the Internet in the illustrated example. The server 7 is a server that is held by a facility such as a hospital and holds basic data such as a medical record of the subject 11 and is connected to the network 5. The server 7 may be a server that is held by an external agency and used by a facility such as a hospital. The occupational therapy support device 101 can acquire basic data such as the age, medical history, and the like of the subject 11 by accessing the server 7. Even in communication between the occupational therapy support device 101 and the server 7, it is possible to prevent information leakage by, for example, requiring the input of an identification code (ID) and a password. Each item of the basic data will also be described later.

The server 9 is connected to the network 5 and constructs an artificial intelligence which can be used via the network 5. The occupational therapy support device 101 estimates activity of daily living data and physical function data based on the sleep data and basic data of the subject 11 of occupational therapy evaluation using the artificial intelligence. The activity of daily living data is data of items about daily activities, such as eating, going to the bathroom, defecation, transfer to the bathroom, locomotion/walk, comprehension, problem solving, and memory. The physical function data is data of items about physical functions such as grip strength. Each item of daily life data and physical function data will also be described later. The artificial intelligence may be constructed in the computer 10 as a part of the occupational therapy support device 101, may be constructed in the computer 10 so that the occupational therapy support device 101 can access the artificial intelligence separately from the occupational therapy support device 101, or may be an artificial intelligence outside the computer 10 like the artificial intelligence provided by the server 9. Even in communication between the occupational therapy support device 101 and the server 9, it is possible to prevent information leakage by, for example, requiring the input of an identification code (ID) and a password.

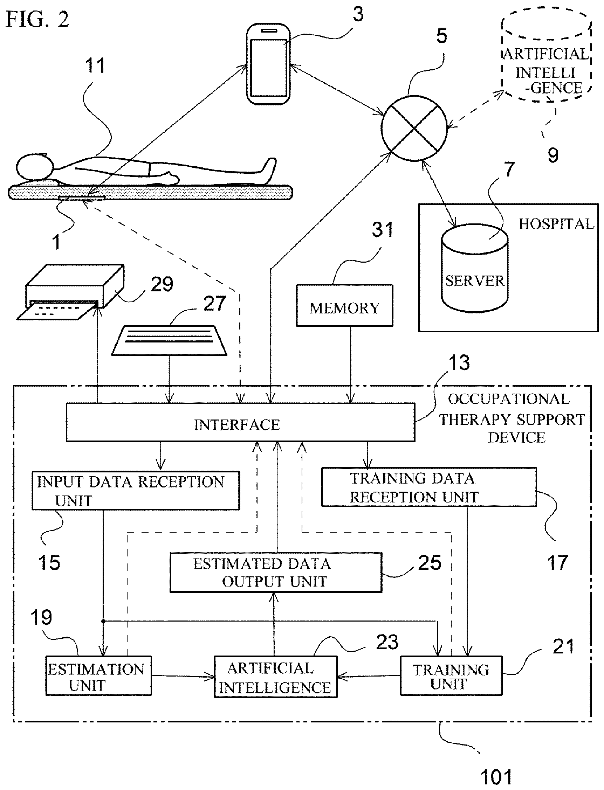

FIG. 2 is a block diagram illustrating the configuration of the occupational therapy support device 101. The occupational therapy support device 101 includes an interface 13, an input data reception unit 15, a training data reception unit 17, an estimation unit 19, a training unit 21, an artificial intelligence 23, and an estimated data output unit 25. The interface 13 is a device portion that enables communication between the occupational therapy support device 101 itself and an external device in accordance with a predetermined protocol for each external device. Communication between the sleep sensor 1, the user communication terminal 3, the servers 7 and 9, an input device 27 such as a keyboard, an output device 29 such as a printer or display, a storage medium 31 such as a USB memory or CDROM, and the occupational therapy support device 101 is performed via the interface 13.

The input data reception unit 15 receives the input of input data including the sleep data of the subject 11 and the basic data of the subject 11. The estimation unit 19 inputs the input data whose input has been received by the input data reception unit 15 to the artificial intelligence 23 to cause the artificial intelligence 23 to compute estimated data regarding the activity of daily living data and the physical function data of the subject 11. If training has already been performed, the artificial intelligence 23 outputs the estimated data with high accuracy concerning the activity of daily living data and the physical function data. The estimated data output unit 25 outputs the estimated data computed by the artificial intelligence 23. The estimated data output by the estimated data output unit 25 is transmitted to, for example, the user communication terminal 3 or the output device 29 via the interface 13. This allows the occupational therapist or the user to obtain the estimated data of the items of occupational therapy evaluation. The occupational therapist who has received the estimated data directly or via the user can prepare an occupational therapy plan based on the received estimated data and perform occupational therapy based on the prepared plan.

The computer 10 (see FIG. 1) in which the occupational therapy support device 101 is incorporated may be provided in, for example, a facility of an occupational therapist, a hospital, or the like, or may be a mobile computer that can be carried by the occupational therapist to the home of the subject 11. When the subject 11 is hospitalized in a facility, a hospital, or the like and the computer 10 is equipment in the facility, the hospital, or the like, it is possible to directly perform communication between the occupational therapy support device 101 and the sleep sensor 1 without via the user communication terminal 3.

The artificial intelligence 23 can output estimated data with high accuracy through machine learning. The occupational therapy support device 101, since including the training data reception unit 17 and the training unit 21, can train the artificial intelligence 23 by itself without using an external artificial intelligence training device. That is, the occupational therapy support device 101 also incorporates an artificial intelligence training device that trains the artificial intelligence 23 as machine learning. When the occupational therapy support device 101 performs machine learning, the input data reception unit 15 receives the input of input data including sleep data and basic data, and the training data reception unit 17 receives the input of training data that is a set of correct activity of daily living data and physical function data corresponding to these input data. The training unit 21 inputs the input data whose input has been received by the input data reception unit 15 and the training data received by the training data reception unit 17 to the artificial intelligence 23 to train the artificial intelligence 23 so as to estimate the training data from the input data. Inputting a large number of sets of input data and training data associated with each other to the occupational therapy support device 101 will foster the training of the artificial intelligence 23 and improve the estimation accuracy.

In the past, the sleep data and the basic data collected for various subjects 11 and the activity of daily living data and the physical function data obtained by actual measurement in correspondence with these data are recorded in the storage medium 31 in association with each other, for example, so that the input data reception unit 15 and the training data reception unit 17 sequentially read a large number of data required for training from the storage medium 31, and the training unit 21 can repeat the training of the artificial intelligence 23 for each read data. As described above, the occupational therapy support device 101 can switch and execute two operation modes, namely, an estimation mode for computing and outputting estimated data using the artificial intelligence 23 and a training mode for training the artificial intelligence 23 as machine learning. For example, the input device 27 can instruct the switching of the operation modes.

In the example of FIG. 2, the artificial intelligence 23 is incorporated in the computer 10 as part of the occupational therapy support device 101. On the other hand, as illustrated by the dotted line in FIG. 2, the artificial intelligence constructed in the external server 9 or the like may be used. In this case, the estimation unit 19, the training unit 21, and the estimated data output unit 25 operate the external artificial intelligence via the network 5 and the like. The estimated data output unit 25 causes the external artificial intelligence to output estimated data, for example, receives the estimated data by the estimated data output unit 25 via the interface 13, and further outputs the received estimated data to the output device 29, the user communication terminal 3, or the like via the interface 13 by the estimated data output unit 25. When the external artificial intelligence is used, the artificial intelligence 23 constituting a part of the occupational therapy support device 101 is unnecessary.

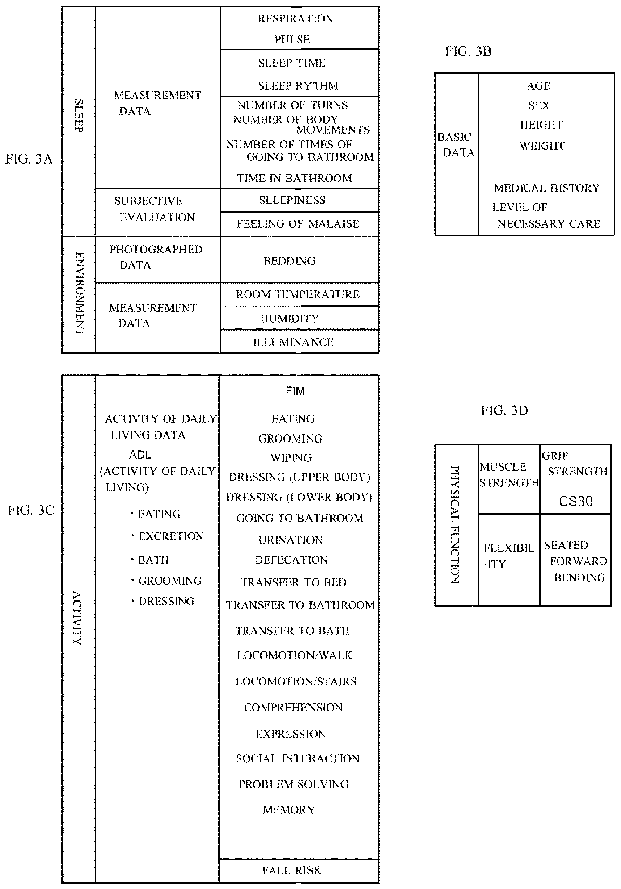

FIGS. 3A to 3D are tabular diagrams illustrating input data and output data of the occupational therapy support device 101. FIG. 3A illustrates sleep data and environment data during sleep, FIG. 3B illustrates basic data, FIG. 3C illustrates activity of daily living data, and FIG. 3D illustrates physical function data. The following will exemplify how to express each data to be handled by the occupational therapy support device 101. Obviously, this is merely an example and other expressions can be used.

Of the sleep data (see FIG. 3A), the sleep time and the time in the bathroom are expressed in units of time (h) as 6.5. The sleep rhythm is represented by a time-series change in sleep time and wakefulness time and is represented by, for example, a data string such as (WAKEFULNESS, WAKEFULNESS, SLEEP, SLEEP, SLEEP, SLEEP, WAKEFULNESS, WAKEFULNESS, SLEEP, ...) indicating whether the sleep state or the wakefulness state is observed at 15 minute intervals from the time of getting into bed to 9 hours later. This makes it possible to obtain the time from lying down to falling asleep, which is an index of good or bad falling asleep. "Sleep" and "wakefulness" are represented by codes assigned in advance, for example, numerical values "1" and "0". The number of turns, the number of body movements, and the number of times of going to the bathroom are represented by natural numbers such as 1, 2, and 3. The number of body movements is the number of movements performed during lying down, excluding turning over, and means, for example, the number of movements such as moving a foot or taking out a hand from a comforter. The number of times of going to the bathroom means the number of times of leaving the bed for going to the bathroom during the sleeping time. Respiration and pulse each are represented by the number of times within one minute. A room temperature, humidity, and illuminance, which are environment data, are represented by numerical values based on the units of temperature, humidity, and illuminance, respectively. The sleep data and the environment data are acquired by the sleep sensor 1. Alternatively, the above sleep data may be generated by the application of the user communication terminal 3 or the application of the computer 10 on the basis of the raw data acquired by the sleep sensor 1. That is, data regarding, for example, lying down, sleep, wakefulness, turning over, and body movement is generated by the sleep sensor 1 itself or the application from a change in pressure, a heart rate, a respiratory rate, and the like sensed by the sleep sensor 1. Even when it is not possible to specify which part of the body has been moved with regard to the body movement, it is possible to detect that the body movement is not rolling movement.

Of the subjective evaluation data included in the sleep data, the sleepiness includes any one of the options namely "had a good sleep", "could not say either", and "did not sleep", and each option is represented by a code assigned in advance, for example, numerical values "1", "2", and "3". Alternatively, each option may be represented by a code corresponding to selection or non-selection, for example, a numerical value such as "1" or "0". The feeling of malaise includes, for example, "pleasant", "average", and "dull". These subjective evaluation data are input, for example, by a user, an occupational therapist, or the like touching an option displayed on the screen of the user communication terminal 3 or the computer 10 in which the application is activated. For example, dialog "Sleep well?" appears on the screen as a question about sleepiness, and "I had a good sleep", "I cannot say either", and "I did not sleep" are simultaneously displayed as options. When the user or the like touches the option "I had a good sleep", a code corresponding to the option "I had a good sleep" is input to the application. This code is input to the occupational therapy support device 101 as an answer to sleepiness.

Of the environment data, the bedding is photographic data of the bedding. The photograph data is photographed by the camera attached to the user communication terminal 3 or the computer 10 in which the application is activated. The image data acquired by photographing is input to the occupational therapy support device 101. The image data is represented by a set of pixel values.

The basic data (see FIG. 3B) is acquired from, for example, the server 7 of the hospital. Alternatively, for example, the information may be manually input to the user communication terminal 3 or the computer 10 that has started the application. Of the basic data, the age, height, and weight are represented by numerical values based on these units. The sex is represented by a code corresponding to a male or a female, for example, a numerical value such as "0" or "1". The medical history is represented by codes assigned in advance to various disease names, for example, numerical values such as "0", "1", "2", .... Alternatively, each disease name may be expressed by a reference sign corresponding to "absent" or "present", for example, a numerical value such as "0" or "1". The degree of care represents the level of necessary care, and is represented by, for example, numerical values in eight levels.

The activity of daily living data (see FIG. 3C) includes 18 items based on the functional independence rating method (FIM), which is known as an effective means of occupational therapy evaluation. For the daily movement of each item, how much the subject 11 can perform by himself/herself is evaluated in seven levels of 1 point to 7 points. The activity of daily living data includes cognitive items and movement items. The cognitive items include five items namely comprehension, expression, social interaction, problem solving, and memory. The movement items include other 13 items namely eating, grooming, wiping, dressing, going to the bathroom, urination management, defecation management, transfer (sitting-up movement), and locomotion. Each item is expressed by a numerical value corresponding to a score. The activity of daily living data further includes "fall risk". A fall risk is obtained by evaluating the possibility of falling and is expressed by, for example, numerical values in two levels namely "0" and "1" corresponding to "high possibility" and low possibility", respectively, or numerical values such as "0", "1", "2", and "3" corresponding to many levels further subdivided.

Of the physical function data (see FIG. 3D), the grip strength as an index of muscle strength is represented by a numerical value (for example, a numerical value in kgw) representing the grip strength. CS30, which is also known as an index of muscle strength, represents how many times a person can stand up from a chair in 30 seconds and is represented by a numerical value indicating the number of times. Of the physical function data, seated forward bending as an index of flexibility represents how much the position of the hands moves forward when the body is bent forward while the arms are extended forward in a long sitting posture and is expressed, for example, in units of centimeters. The estimated data output unit 25 converts the estimated values computed by the artificial intelligence 23, regarding the activity of daily living data expressed by discontinuous numerical values in seven levels, into numerical values each closest to one of the numerical values in seven levels by, for example, rounding off and outputs the converted values. Since the data after the conversion is based on the estimated data of the activity of daily living data computed by the artificial intelligence 23, the data after the conversion is still the estimated data of the activity of daily living data.

In order to solve the problem of obtaining an objective occupational therapy evaluation that does not depend on the evaluator, the inventor of the present application having long-time experience as an occupational therapist has conceived to estimate activity of daily living data and physical function data on the basis of sleep data and basic data that are objective data that do not depend on an evaluator. It was expected that there was a complex but correlated relationship between a set of sleep data and basic data and a set of activity of daily living data and physical function data. Therefore, the present inventor has considered that it would be possible in principle to estimate activity of daily living data and physical function data on the basis of the sleep data and the basic data even if it is an excessive burden and is not realistic to perform such estimation by human intelligence. Then, it has been conceived that acquisition of such estimated data of occupational therapy evaluation beyond human intelligence can be made realistic by using an artificial intelligence. Since sleep data and basic data as the foundation are objective data that do not depend on the evaluator, the obtained activity of daily living data and the estimated data of physical function data are also objective data that do not depend on the evaluator.

On the basis of the experience of the occupational therapist, it is sufficient to have data of items namely eating, going to the bathroom, defecation, transfer to the bathroom, locomotion/walk, comprehension, problem solving, and memory as activity of daily living data in order to perform occupational therapy evaluation as a premise for drafting an occupational therapy plan. If there is data of an item of grip strength as physical function data and there are data of more items, more accurate occupational therapy evaluation can be obtained. Similarly, based on the experience of the occupational therapist, in order to obtain the minimum items of the above-described occupational therapy evaluation, data of items namely the number of turns, the number of body movements, the number of times of going to the bathroom, and the time in the bathroom is sufficient as sleep data, and data of the items namely age, height, weight, and medical history is sufficient as basic data. Conversely, even when the number of items of activity of daily living data, sleep data, and basic data is smaller than the number of items described above, the obtained estimated data can be useful for occupational therapy evaluation. Obtained estimated data is objective data that does not depend on the evaluator as long as it is based on objective data that does not depend on the evaluator, regardless of whether the number of items is large or small.

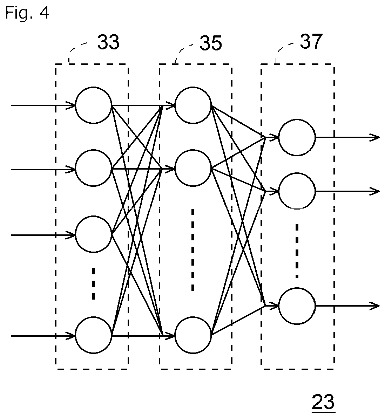

FIG. 4 is a schematic diagram illustrating the conceptual configuration of the artificial intelligence 23 used by the occupational therapy support device 101. The artificial intelligence provided by the server 9 has a similar configuration as an example. The illustrated artificial intelligence 23 is a neural network and includes an input layer 33 in which nodes receiving the input of data are arranged, an output layer 37 in which nodes outputting operation result data are arranged, and an intermediate layer 35 in which nodes connecting the input layer 33 and the output layer 37 are arranged. In the illustrated example, the intermediate layer 35 is single, but may span multiple layers. The value of the previous node is transmitted to the next node while reflecting the parameter given to each node, that is, the weight and the bias value of each node. The input layer 33 receives the input data whose input has been received by the input data reception unit 15, that is, a set of items of sleep data and basic data. The input data is transmitted to the output layer 37 via the intermediate layer 35 while reflecting the parameter of each node. The data transmitted to the output layer 37 is the estimated data of a set of items of the activity of daily living data and the physical function data. The estimation unit 19 (see FIG. 2) inputs a set of items of the sleep data and the basic data of the subject 11 to the input layer 33 of the artificial intelligence 23 and causes the output layer 37 to generate the activity of daily living data and the estimated data of the physical function data of the subject 11. The estimated data output unit 25 outputs the generated estimated data after performing conversion such as rounding off or without performing conversion.

In order for estimated data appearing at a node of the output layer 37 to be the estimated data of the activity of daily living data and the physical function data with high accuracy, it is necessary to train the artificial intelligence 23 using the actually measured activity of daily living data and physical function data. Training is performed by inputting, to the input layer 33, a set of items of the sleep data and the basic data of a certain subject 11 whose input has been received by the input data reception unit 15, and inputting, to the output layer 37, training data about the same subject 11 received by the training data reception unit 17, that is, a set of items of the measured activity of daily living data and the physical function data as training data. The training unit 21 (see FIG. 2) inputs such data to the artificial intelligence 23.

The artificial intelligence 23 computes the estimated data of activity of daily living data and physical function data based on the input sleep data and the basic data, generates the estimated data in the output layer 37, and computes an error between the generated estimated data and the activity of daily living data and the physical function data input as training data. The artificial intelligence 23 then changes the parameter of each node from the output layer 37 toward the input layer 33 by, for example, a well-known error back propagation algorithm so as to generate estimated data without error. Such a function is included in the artificial intelligence 23 itself. Preparing a large number of sets of input data and training data and repeating training will cause the artificial intelligence 23 to compute estimated data with high accuracy. When the artificial intelligence 23 is trained, the number of intermediate layers 35 and the number of nodes of each layer can be adjusted to optimum values. Such techniques are also well known.

In order to obtain the estimated data of the activity of daily living data and the physical function data of the subject 11, it is possible to input not only the latest data to the artificial intelligence 23, concerning the sleep data and the basic data of the subject 11, but also input data at a plurality of time points including data before the latest data to the artificial intelligence 23 together with the time data of each data. As a result, regarding the sleep data and the basic data of the subject 11, the estimated data of the activity of daily living data and the physical function data of the same subject 11 are obtained in consideration of also the past history. This makes it possible to obtain estimated data with higher accuracy. Time data may be represented by, for example, the date and time at each time point or may be represented by a date and time difference from the latest time point. The input data reception unit 15 receives data at a plurality of time points together with the respective time data, and the estimation unit 19 inputs the data at the plurality of time points and the respective time data to the input layer 33 of the artificial intelligence 23. The larger the number of time points of the input data, the more the number of nodes of the input layer 33 receiving the input of the data increases in proportion thereto.

In order to obtain estimated data based on data at a plurality of time points, it is necessary to train the artificial intelligence 23 based on the data at the plurality of time points, the respective time data, and the training data corresponding to the respective data. For example, in order to obtain the estimated data of activity of daily living data and physical function data from sleep data and basic data at past three time points including the latest time point, the artificial intelligence 23 can be trained by inputting the sleep data and the basic data at the three time points of various subjects 11 and the respective time data to the input layer 33 and inputting the latest actual measurement data of the activity of daily living data and the physical function data of each subject 11 to the output layer 37. Since the time data is simultaneously input, a plurality of time points at which sleep data and basic data are collected may be different among different subjects 11. For example, data at the latest time point, 1 week ago, and 5 weeks ago may be input for a certain subject 11, and data at the latest time point, 3 weeks ago, and 15 weeks ago may be input for another subject. The artificial intelligence 23 adjusts the parameter of the node so as to compute estimated data reflecting the influence of the temporal distance from the latest time point through training with a large number of data.

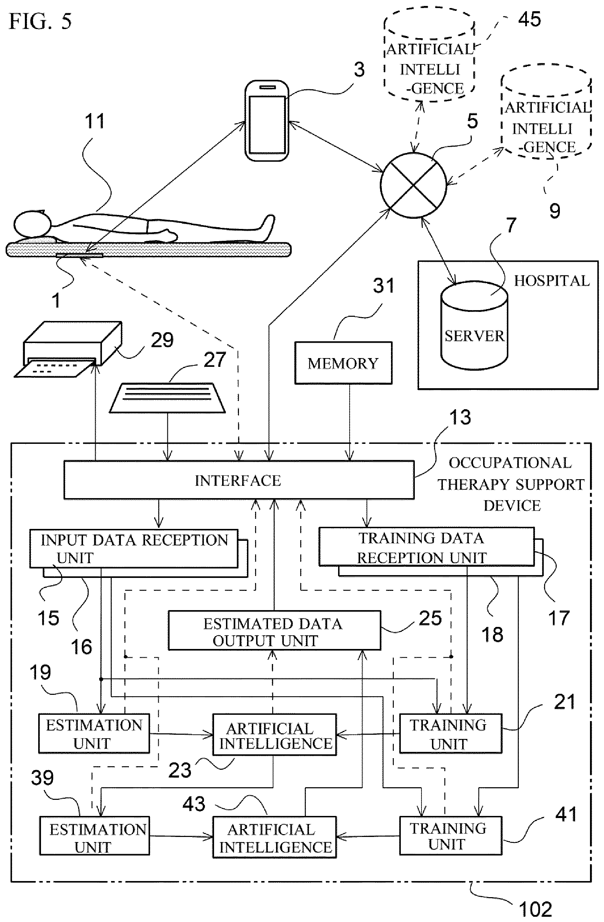

FIG. 5 is a block diagram illustrating the configuration of an occupational therapy support device according to another embodiment of the invention. An occupational therapy support device 102 is different from the occupational therapy support device 101 (see FIG. 2) in further including another input data reception unit 16, another training data reception unit 18, another estimation unit 39, another artificial intelligence 43, and another training unit 41. In the occupational therapy support device 102, the estimation unit 39 reads out the estimated data computed by the artificial intelligence 23, that is, the estimated data regarding the activity of daily living data and the physical function data of the subject 11. The estimation unit 39 inputs the read estimated data of the artificial intelligence 23 to the artificial intelligence 43, thereby causing the artificial intelligence 43 to compute the estimated data regarding the prescription data expressing the information to be prescribed to the subject 11. Similar to the estimated data output unit 25, the estimation unit 39 inputs the read estimated data of the artificial intelligence 23 to the artificial intelligence 43 upon converting the estimated data by rounding off or the like or without conversion.

If training has already been performed, the artificial intelligence 43 outputs estimated data with high accuracy regarding prescription data. The estimated data output unit 25 converts the estimated data computed by the artificial intelligence 43 or outputs the estimated data without conversion. The estimated data output by an estimated data output unit 45 is transmitted to, for example, the user communication terminal 3 or the output device 29 via the interface 13. As a result, the occupational therapist or the user can obtain estimated data regarding the contents of the occupational therapy to be prescribed. The occupational therapist who has received the estimated data of prescription data directly or through the user can use the received estimated data for planning an occupational therapy plan and perform occupational therapy.

As described above, since the sleep data and the basic data of the subject 11 are objective data that do not depend on the evaluator, the estimated data computed by the artificial intelligence 23 on the basis of the sleep data and the basic data, that is, the estimated data regarding the activity of daily living data and the physical function data of the subject 11 are also objective data that do not depend on the evaluator. Similarly, since the estimated data regarding the activity of daily living data and the physical function data of the subject 11 are objective data that do not depend on the evaluator, the estimated data computed by the artificial intelligence 43 based on the estimated data, that is, the estimated data regarding the prescription data for the subject 11 are also objective data that do not depend on the evaluator. That is, according to the occupational therapy support device 102, the estimated data useful for the occupational therapy evaluation is obtained as the objective data independent of the evaluator regardless of whether or not the estimated data is output to the outside, and the estimated data useful for prescribing the occupational therapy is obtained as the objective data independent of the evaluator based on the estimated data. Note that the estimated data output unit 25 may output not only the estimated data regarding the prescription data of the subject 11 computed by the artificial intelligence 43 but also the estimated data computed by the artificial intelligence 23, that is, the estimated data regarding the activity of daily living data and the physical function data of the subject 11 to the outside via the interface 13, similarly to the occupational therapy support device 101.

Similarly to the artificial intelligence 23, the artificial intelligence 43 can output estimated data with high accuracy via machine learning. The occupational therapy support device 102 includes the input data reception unit 16, the training data reception unit 18, and the training unit 41, so that the occupational therapy support device 102 itself can train the artificial intelligence 43 without using an external artificial intelligence training device. That is, the occupational therapy support device 102 also incorporates an artificial intelligence training device that trains the artificial intelligence 43 as machine learning. When the occupational therapy support device 102 trains the artificial intelligence 43, the input data reception unit 16 receives the input of the input data including the activity of daily living data and the physical function data, and the training data reception unit 18 receives the input of the training data which is the correct prescription data corresponding to these input data. The training unit 41 inputs the input data whose input has been received by the input data reception unit 16 and the training data received by the training data reception unit 18 to the artificial intelligence 43 to train the artificial intelligence 43 so as to estimate the training data from the input data. Inputting a large number of sets of input data and training data associated with each other to the occupational therapy support device 102 will foster the training of the artificial intelligence 43 and improve the estimation accuracy.

In the past, the activity of daily living data and the physical function data collected for various subjects 11 and the prescription data indicating the prescription of the proven occupational therapy performed in correspondence with these data or the correct prescription to be performed are recorded in the storage medium 31 in association with each other, for example, so that the input data reception unit 16 and the training data reception unit 18 sequentially read a large number of data required for training from the storage medium 31, and the training unit 41 can repeat the training of the artificial intelligence 43 for each read data. As described above, the occupational therapy support device 102 can switch and execute two operation modes, namely, an estimation mode for computing and outputting estimated data using the artificial intelligence 43 and a training mode for training the artificial intelligence 43 as machine learning. For example, the input device 27 can instruct the switching of the operation modes. In the example of FIG. 5, the artificial intelligence 43 is incorporated in the computer 10 as part of the occupational therapy support device 102. On the other hand, as illustrated by the dotted line in FIG. 5, the artificial intelligence constructed in the external server 45 or the like may be used. In this case, the estimation unit 39, the training unit 41, and the estimated data output unit 25 operate the external artificial intelligence via the network 5 and the like. When external artificial intelligence is used, the artificial intelligence 43 constituting a part of the occupational therapy support device 102 is unnecessary.

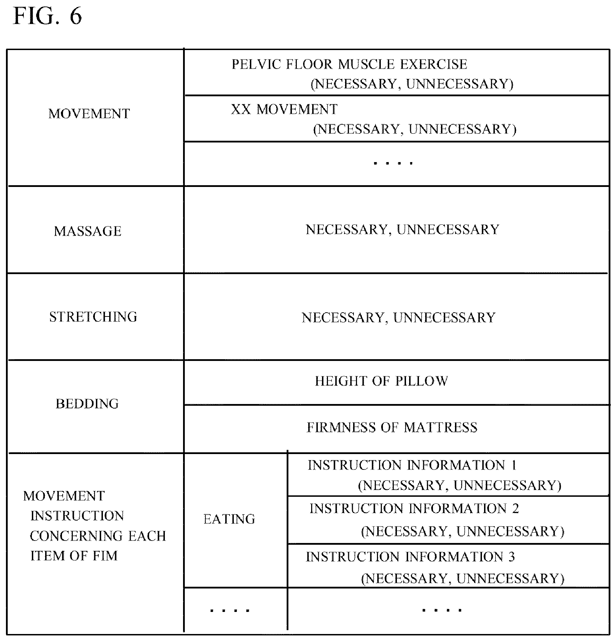

FIG. 6 is a tabular diagram illustrating prescription data that is output data of the occupational therapy support device 102. In the illustrated example, the prescription data includes prescription information for movement, massage, stretching, bedding conditions, and movement instruction information for each item of FIM. Regarding movement, for example, the prescription data includes various types of movement items in addition to pelvic floor muscle exercise, and the prescription data expresses whether or not the movement of each item is necessary. For example, when there are five types of movement items, prescription data regard movement is configured by a set of data indicating necessity such as (necessary, necessary, unnecessary, unnecessary, unnecessary). "Necessary" and "unnecessity" are represented by, for example, numerical values such as "1" and "0". The prescription data regarding massage represents whether or not massage is necessary. The prescription data regarding stretching likewise represents whether stretching is necessary. The prescription data for bedding is, in the illustrated example, the height of a pillow and the firmness of a mattress. Each prescription data is expressed by, for example, a numerical value based on a predetermined unit. In addition to "necessary" and "unnecessary", the information regarding movement, massage, and stretching may include body parts used for movement and the like, time, the number of times, necessity of a break to be taken between movements or the like, and a break time.

Regarding the movement instruction for each item of FIM, several pieces of instruction information are prepared in advance for each item. For example, if three types of instruction information 1, 2, and 3 are prepared for each item, prescription data containing movement instructions for all the 18 items of FIM is constituted by a set of 18 pieces of instruction information such as (2, 1, 3, ..., 2). Each instruction information is represented by, for example, a numerical value such as "1", "2", or "3". Alternatively, for example, "necessary" or "unnecessary" may be selected for each of the three types of movement information for each item of FIM. For example, selecting movement instructions such as (necessary, necessary, unnecessary) constitute prescription data containing a movement instruction for one item of FIM. Arranging such 18 prescription data will constitute prescription data containing movement instructions for all the 18 items of FIM. "Necessary" and "unnecessity" are represented by, for example, numerical values such as "1" and "0". The estimated data output unit 25 converts the estimated value computed by the artificial intelligence 43, regarding the items of prescription data which are expressed by discontinuous numerical values such as "0", "1", "2", ... into numerical values each closest to one of the discontinuous numerical values by, for example, rounding off and outputs the converted values. When estimated data of (necessary, necessary, unnecessary) is obtained for a plurality of items that are not compatible with each other and should be selected alone, it is also possible to select an item closest to the code of "necessary", for example, "1". As an example of instruction information regarding the item "eating" illustrated in FIG. 6, the following are assumed: "suppress water intake" as instruction information 1 and "change drink from green tea to water" as instruction information 2. In these examples, when the sleep data indicates that the number of times of going to the bathroom is large, it is predicted that there is a high possibility of being "necessary".

The estimated data of the prescription data output by the occupational therapy support device 102 may not include all the items exemplified in FIG. 6 but may include only some of them, for example, only one item. Even with only some items, the estimated data of obtained prescription data can be useful for prescribing occupational therapy. If the estimated data of the prescription data output by the occupational therapy support device 102 is sufficient to include only some items, it is sufficient to use only some items as training data also in the training of the artificial intelligence 43.

The description referring to FIGS. 3A to 3D and 6 has exemplified the form in which each item included in each of the sleep data, the basic data, the activity of daily living data, the physical function data, and the prescription data is expressed by a code associated in advance such as a numerical value. Code representation is convenient for the artificial intelligences 23 and 43 to handle. When each data is output to the output device 29 such as a display, it is desirable that each data is output not in the form of a message that can be read by the user, the occupational therapist, or the like, such as a sentence instead of a code without any change. Obviously, a code can be easily converted into a message form by an application of the user communication terminal 3 or the estimated data output unit 25 of the occupational therapy support device 101 or 102. Even if the data is converted into such a message form, the data is still estimated data.

In the above description, an occupational therapist has been described as an example of a person who uses the estimated data output from the occupational therapy support devices 101 and 102. However, a person who performs occupational therapy using estimated data is not limited to an occupational therapist and may be, for example, a user including the subject 11 or may be another medical or care worker such as a doctor, a physical therapist, or a care worker. In particular, there is an advantage that the estimated data of the prescription data output by the occupational therapy support device 102 is easily used by general users.

This application is based on Japanese Patent Application No. 2020 024812 filed by the applicant in Japan on February 18, 2020.

### REFERENCE SIGNS LIST

- 1: sleep sensor
- 3: user communication terminal
- 5: network
- 7, 9: server
- 10: computer
- 11: subject
- 13: interface
- 15, 16: input data reception unit
- 17, 18: training data reception unit
- 19: estimation unit
- 21: training unit
- 23: artificial intelligence
- 25: estimated data output unit
- 27: input device
- 29: output device
- 31: memory
- 33: input layer
- 35: intermediate layer
- 37: output layer
- 39: estimation unit
- 41: training unit
- 43: artificial intelligence
- 100: occupational therapy assistance system
- 101, 102: occupational therapy support device.

## Claims

1. An occupational therapy support device (101, 102) comprising:
an input data reception unit (15) that receives input data including
(i) sleep data regarding sleep of a subject (11) of occupational therapy evaluation and
(ii) basic data regarding a body of the subject (11);
a first estimation unit (19) that inputs the input data received by the input data reception unit to a trained first artificial intelligence (23) to cause the first artificial intelligence to compute estimated data including data regarding activity of daily living of the subject (11);
a first estimated data output unit (25) that outputs the estimated data computed by the first artificial intelligence (23),
a second estimation unit (39) that inputs the estimated data computed by the first artificial intelligence (23) to a trained second artificial intelligence (43) to cause the second artificial intelligence (43) to compute estimated data of prescription data of occupational therapy for the subject (11); and
a second estimated data output unit (45) that outputs estimated data of the prescription data computed by the second artificial intelligence (43),wherein
the sleep data includes data that is based on data measured by a sleep sensor (1) and expressed in a numerical value,
the basic data includes data expressed in a numerical value,
the data regarding activity of daily living includes data that is evaluated stepwise in a numerical value,
the data regarding activity of daily living includes at least part of evaluation items defined in Functional Independence Measure, FIM, and
the prescription data includes data expressing contents to be prescribed for at least one item of movement, massage, stretching, and a bedding condition.

2. The occupational therapy support device (101) according to claim 1, wherein the prescription data further includes data expressing a movement instruction for at least one item of activity of daily living included in the data regarding activity of daily living.

3. The occupational therapy support device (101) according to any one of claims 1 or 2, wherein,
the sleep data includes at least part of respiration, pulse, sleep time, sleep rhythm, the number of turns, the number of body movements, the number of times of going to a bathroom, and time in the bathroom, and
the basic data including at least part of age, sex, height, weight, medical history, and a level of necessary care.

4. The occupational therapy support device (101) according to any one of claims 1 to 3, wherein the data regarding activity of daily living includes a fall risk which is a possibility of falling.

5. The occupational therapy support device (101) according to any one of claims 1 to 4, wherein the input data further includes environment data regarding an environment of the subject (11) during sleep.

6. The occupational therapy support device (101) according to any one of claims 1 to 5, wherein the input data includes the sleep data of the subject (11) and the basic data of the subject (11) at a plurality of time points from a present to a past in association with time data of the plurality of corresponding time points.

## Patentansprüche

1. Ergotherapieunterstützungsvorrichtung (101, 102), umfassend:
- eine Eingabedaten-Empfangseinheit (15), die Eingabedaten empfängt, die Folgendes umfassen:
(i) Schlafdaten betreffend den Schlaf eines Probanden (11) der Ergotherapeutischen Beurteilung und
(ii) Basisdaten betreffend den Körper des Probanden (11);
- eine erste Schätzeinheit (19), die die von der Eingangsdaten-Empfangseinheit empfangenen Eingangsdaten an eine trainierte erste künstliche Intelligenz (23) übermittelt, um die erste künstliche Intelligenz dazu zu veranlassen, Schätzwerte zu berechnen, einschließlich Daten betreffend die Aktivitäten des täglichen Lebens des Probanden (11);
- eine erste Schätzwert-Ausgabeeinheit (25), die die von der ersten künstlichen Intelligenz (23) berechneten Schätzwerte ausgibt,
- eine zweite Schätzeinheit (39), die die von der ersten künstlichen Intelligenz (23) berechneten Schätzwerte an eine trainierte zweite künstliche Intelligenz (43) übergibt, um die zweite künstliche Intelligenz (43) dazu zu veranlassen, Schätzwerte für Ergotherapie-Verschreibungsdaten für den Probanden (11) zu berechnen, und
- eine zweite Schätzwerte-Ausgabeeinheit (45), die die von der zweiten künstlichen Intelligenz (43) berechneten Schätzwerte der Verschreibungsdaten ausgibt, wobei
die Schlafdaten Daten umfassen, die auf von einem Schlafsensor (1) gemessenen Daten basieren und als ein numerischer Wert ausgedrückt werden,
die Basisdaten Daten umfassen, die als ein numerischer Wert ausgedrückt sind, die Daten betreffend Aktivitäten des täglichen Lebens Daten umfassen, die schrittweise anhand eines numerischen Werts bewertet werden,
die Daten betreffend Aktivitäten des täglichen Lebens zumindest einen Teil der im Functional Independence Measure, FIM, definierten Bewertungskriterien umfassen, und
die Verschreibungsdaten Daten umfassen, die Verschreibungsinhalte für zumindest eines der Elemente Bewegung, Massage, Dehnung und Schlafstellenbeschaffenheit ausdrücken.

2. Ergotherapieunterstützungsvorrichtung (101) nach Anspruch 1,
wobei die Verschreibungsdaten ferner Daten umfassen, die eine Bewegungsanweisung für zumindest ein Element der Aktivitäten des täglichen Lebens wiedergeben, die in den Daten betreffend die Aktivitäten des täglichen Lebens erhalten sind.

3. Ergotherapieunterstützungsvorrichtung (101) nach einem der Ansprüche 1 oder 2, wobei
die Schlafdaten zumindest einen Teil von Atmung, Puls, Schlafzeit, Schlafrythmus, Anzahl an Drehungen, Anzahl an Körperbewegungen, Anzahl an Toilettengängen und Zeit im Badezimmer umfassen, und
die Basisdaten zumindest einen Teil von Alter, Geschlecht, Größe, Gewicht, Krankengeschichte und eines erforderlichen Pflegebedarfs umfassen.

4. Ergotherapieunterstützungsvorrichtung (101) nach einem der Ansprüche 1 bis 3, wobei die Daten betreffend die Aktivitäten des täglichen Lebens ein Sturzrisiko umfassen, bei dem es sich um eine Möglichkeit eines Stürzens handelt.

5. Ergotherapieunterstützungsvorrichtung (101) nach einem der Ansprüche 1 bis 4, wobei die Eingabedaten ferner Umgebungsdaten betreffend eine Umgebung des Probanden (11) während des Schlafs umfassen.

6. Ergotherapieunterstützungsvorrichtung (101) nach einem der Ansprüche 1 bis 5, wobei die Eingabedaten die Schlafdaten des Probanden (11) und die Basisdaten des Probanden (11) zu einer Vielzahl von Zeitpunkten von einem gegenwärtigen Zeitpunkt bis zu einem vergangenen Zeitpunkt in Verbindung mit Zeitdaten der Vielzahlzahl entsprechender Zeitpunkte umfassen.

## Revendications

1. Dispositif d'assistance à l'ergothérapie (101, 102) comprenant :
• une unité de réception de données d'entrée (15) qui reçoit des données d'entrée comprenant :
∘ (i) des données de sommeil concernant le sommeil d'un sujet (11) d'une évaluation d'ergothérapie, et
∘ (ii) des données de base concernant le corps du sujet (11) ;
• une première unité d'estimation (19) qui introduit les données d'entrée reçues par l'unité de réception de données d'entrée dans une première intelligence artificielle entraînée (23) pour amener la première intelligence artificielle à calculer des données estimées comprenant des données concernant l'activité de la vie quotidienne du sujet (11) ;
• une première unité de sortie de données estimées (25) qui fournit en sortie les données estimées calculées par la première intelligence artificielle (23) ;
• une seconde unité d'estimation (39) qui introduit les données estimées calculées par la première intelligence artificielle (23) dans une seconde intelligence artificielle entraînée (43) pour amener la seconde intelligence artificielle (43) à calculer des données estimées de données de prescription d'ergothérapie pour le sujet (11) ; et
• une seconde unité de sortie de données estimées (45) qui fournit en sortie des données estimées des données de prescription calculées par la seconde intelligence artificielle (43), dans lequel :
∘ les données de sommeil comprennent des données qui sont basées sur des données mesurées par un capteur de sommeil (1) et exprimées sous forme de valeur numérique,
∘ les données de base comprennent des données exprimées sous forme de valeur numérique,
∘ les données concernant l'activité de la vie quotidienne comprennent des données qui sont évaluées par étapes sous forme de valeur numérique,
∘ les données concernant l'activité de la vie quotidienne comprennent au moins une partie d'éléments d'évaluation définis dans la mesure de l'indépendance fonctionnelle (MIF / *FIM*), et
∘ les données de prescription comprennent des données exprimant des contenus à prescrire pour au moins un élément parmi le mouvement, le massage, l'étirement et une condition de literie.

2. Dispositif d'assistance à l'ergothérapie (101) selon la revendication 1, dans lequel les données de prescription comprennent en outre des données exprimant une instruction de mouvement pour au moins un élément d'activité de la vie quotidienne inclus dans les données concernant l'activité de la vie quotidienne.

3. Dispositif d'assistance à l'ergothérapie (101) selon l'une quelconque des revendications 1 ou 2, dans lequel :
• les données de sommeil comprennent au moins une partie parmi la respiration, le pouls, le temps de sommeil, le rythme de sommeil, le nombre de retournements, le nombre de mouvements corporels, le nombre de fois où le sujet se rend aux toilettes, et le temps passé dans les toilettes, et
• les données de base comprennent au moins une partie parmi l'âge, le sexe, la taille, le poids, les antécédents médicaux, et un niveau de soins nécessaires.

4. Dispositif d'assistance à l'ergothérapie (101) selon l'une quelconque des revendications 1 à 3, dans lequel les données concernant l'activité de la vie quotidienne comprennent un risque de chute qui est une possibilité de chuter.

5. Dispositif d'assistance à l'ergothérapie (101) selon l'une quelconque des revendications 1 à 4, dans lequel les données d'entrée comprennent en outre des données d'environnement concernant un environnement du sujet (11) pendant le sommeil.

6. Dispositif d'assistance à l'ergothérapie (101) selon l'une quelconque des revendications 1 à 5, dans lequel les données d'entrée comprennent les données de sommeil du sujet (11) et les données de base du sujet (11) à une pluralité de points temporels allant d'un moment présent à un moment passé, en association avec des données temporelles de la pluralité de points temporels correspondants.
